# EUROPEAN PATENT APPLICATION

(11) **EP 3 050 513 A1**
(43) Date of publication of application: **03.08.2016**
(21) Application number: 15152736.3
(22) Date of filing: 27.01.2015
(51) Int. Cl.: A61B 7/00, A61B 5/087

(54) **Expiratory flow rate monitoring**

(71) Applicant: Royal College of Art, London SW7 2EU (GB)
(72) Inventor: Kecman, Maja, London, SW7 2EU (GB); Lu, Ifung, London, SW7 2EU (GB); Heys, Simon, London, SW7 2EU (GB); Fusari, Gianpaolo, London, SW7 2EU (GB); Harrison, Matthew James Clear, London, SW7 2EU (GB)
(74) Representative: Lawrence, John

(57) **Abstract**

A method of measuring expiratory flow rate of a subject using a whistle (20) is disclosed. The method comprising: detecting a sound generated when the subject blows into the whistle (20); and determining the expiratory flow rate (65, 93) of the subject using a frequency of the detected sound. A computer program (40) operable, when run on a detection device (30), to cause the detection device (30) to operate according to the method is also disclosed.

## Description

### FIELD OF THE INVENTION

The present invention relates to methods and systems for monitoring expiratory flow rate, for example, peak expiratory flow rate. The methods and systems described herein are particularly useful in the management of asthma, for example as an alternative to conventional peak expiratory flow meters or spirometers, but also have non-diagnostic uses such as guidance for breathing or relaxation exercises.

### BACKGROUND TO THE INVENTION

It is estimated that there are approximately 5.5 million asthma sufferers of varying severities in the UK. Typically, the treatment of asthma involves prescription of medication as well as the identification and avoidance of asthma triggers, such as allergens, exercise, pollution or foods. For some asthma sufferers, ongoing monitoring of lung function is important for effective asthma control. Such ongoing monitoring is typically achieved by taking regular peak expiratory flow rate measurements, for example once a week, or more regularly such as once or twice a day. These peak expiratory flow rate measurements are typically recorded in a written peak flow record, in which the patient makes a daily note of their measured peak expiratory flow rate, as well as their medication use, asthma flare ups and any associated triggers.

Peak expiratory flow rate is a measurement of the volumetric flow a patient can blow out of their lungs in one breath. To obtain a peak flow measurement, a patient inhales deeply, and then blows out sharply into a peak flow measuring device. The most common such measuring device includes a mechanical spring-loaded piston which is displaced by the patient's exhalation. A clinician, or the patient himself, can use a scale provided on the side of the device to read how far the piston has been displaced, which is indicative of the peak flow.

Other known peak flow monitoring devices include threshold devices, such as the whistle disclosed in WO96/37147. This device includes a leak hole of variable size, such that the size of the leak hole can be set to ensure that a critical air flow rate through the device is needed to make a sound: if the exhalation is at a flow rate above the set threshold the device will make a sound, indicating that the pre-set peak flow rate has been achieved, whilst if the exhalation is at a flow rate below the set threshold the device will make no sound.

It has been noted that good patient compliance with an asthma management regime can result in a patient having good control of their asthma, as well as a good understanding of their personal asthma triggers. Conversely, poor compliance with an asthma management regime can result in poor patient understanding of their triggers, unexpected flare-ups and deterioration, which in extreme cases can lead to hospitalisation. However, current asthma management tools tend to have relatively low patient compliance, especially among children. It is therefore an object of the invention to provide an improved system and method for management of asthma. The system is also useful for measuring expiratory flow rate in other applications, such as exercise and relaxation.

### SUMMARY OF THE INVENTION

According to a first aspect of the invention, a method of measuring expiratory flow rate of a subject using a whistle comprises:
detecting a sound generated when the subject blows into the whistle; and
determining the expiratory flow rate of the subject using a frequency of the detected sound.

We have found that the frequency of sound produced by a whistle is dependent on the volumetric flow rate through the whistle. This allows us to determine characteristics of a subject's expiratory flow rate, such as peak expiratory flow, by detecting the frequency of the sound produced.

The method may include comparing the detected frequency to stored calibration data in order to determine the expiratory flow rate of the subject, for example, the peak expiratory flow rate. The calibration data may comprise data relating the flow rate through the whistle to the frequency of sound produced by the whistle, such as a look-up table of historical readings, or a mathematical relationship.

The method may further comprise determining an expected peak flow rate for the subject using average peak flow rate data and user information data. For example, the average peak flow rate data may comprise average data recorded from a cross section of healthy patients of known age, height and sex. The user information data may comprise the subject's age, height and sex.

The method may comprise comparing the detected peak expiratory flow rate of the subject with the expected peak expiratory flow rate in order to generate ranking data for the subject, which might be, for example, a percentage of the expected peak flow rate.

The sound may be detected in a detection device which is separate from the whistle. The detection device might conveniently be a mobile phone or other handheld device including a microphone or having an input for a microphone, such as a heart rate monitor or games console.

The detection device preferably comprises a display, and the method may further comprise displaying a visual indication of the frequency of the detected sound on the display whilst the expiratory flow rate test is in progress. The visual indication may comprise animation, such as an interactive game, and may include a visual indication of the ranking data.

The determined peak expiratory flow rate may be stored in a memory of the detection device. Environmental data may be associated with the determined peak expiratory flow rate, and the method may comprise storing the associated environmental data in the memory of the detection device. Environmental data might be, for example, the date and time at which the peak expiratory flow rate data was detected, and/or may be information on a location in which the peak expiratory flow rate was detected, such as temperature, pollen count, and/or humidity.

The detection device may comprise a transceiver, and the method may further comprise transmitting the determined peak expiratory flow rate to a remote location. Such a remote location might include a remote computer, for example the subject's own computer, the subject's parent's computer, and/or the subject's clinician's computer.

The method may comprise determining a plurality of values for the subject's peak expiratory flow rate in a single test session, and selecting the highest of the plurality of values as the peak expiratory flow rate value for that test session.

The method may further comprise generating calibration data, wherein the calibration data is generated by blowing air through the whistle at a plurality of known flow rates, and, for each flow rate, detecting a frequency of sound produced by the whistle at that flow rate.

The method may further comprise generating an alarm indicating that a test is advised. For example, the system may advise the subject that a scheduled test is due. Alternatively, the system may advise the subject that environmental data indicate a test is advised (e.g. because temperature, humidity and/or pollen count indicate that an attack might be likely). The alarm might be based on time set by someone (e.g. the patient/their clinician, based on someone (e.g. the patient's doctor) remotely looking at data and wanting more measurements, or based on analysing previous data.

According to a further aspect of the invention, there is provided a computer program operable, when run on a detection device, to cause the detection device to operate in accordance with the method described above.

A whistle and detection device for use with the above method are also described.

The invention described herein provides an extremely convenient alternative to existing methods for monitory expiratory flow rate. Most people use a mobile phone or have access to one at home, whilst the whistle is small and light and very easy to carry around and can even be put on a key ring if required. The system described herein is less cumbersome than conventional peak flow meters, and inexpensive - for most, a whistle is the only additional piece of equipment they will need. Furthermore, the system described herein is destigmatizing. Using current peak flow meters outside of the home may deter use, particularly when readings are taken frequently (e.g. if the subject is monitoring responses to therapy and acute attacks).

All of the above advantages encourage better asthma management by encouraging the patient to engage with their asthma monitoring. The interface is designed to engage with children, particularly young children under 5. Adults will also like the ability to interact with an application, rather than simply recording data in a log, as well as to have the ability to easily review their asthma history. The system also makes it more straightforward for clinicians to see a history of all readings, including those taken which are not recorded as the highest peak flow measure in any one session.

### BRIEF DESCRIPTION OF THE DRAWINGS

An embodiment of the invention will now be described, by way of example only, with reference to the accompanying drawings, in which:
**Figure 1** shows an asthma management system;
**Figure 2** shows an example relationship between peak flow rate and frequency;
**Figure 3** shows an example graphic user interface;
**Figure 4** shows further screens from the graphic user interface of Figure 3, including profile, summary and record screens;
**Figure 5** shows further screens from the graphic user interface of Figure 3, including a challenge providing a visual indication of peak flow;
**Figure 6** shows further screens from the graphic user interface of Figure 3, including episode logging screens;
**Figure 7** schematically shows an asthma monitoring application; and
**Figure 8** shows a flow chart setting out a method of asthma monitoring.

### DESCRIPTION OF THE INVENTION

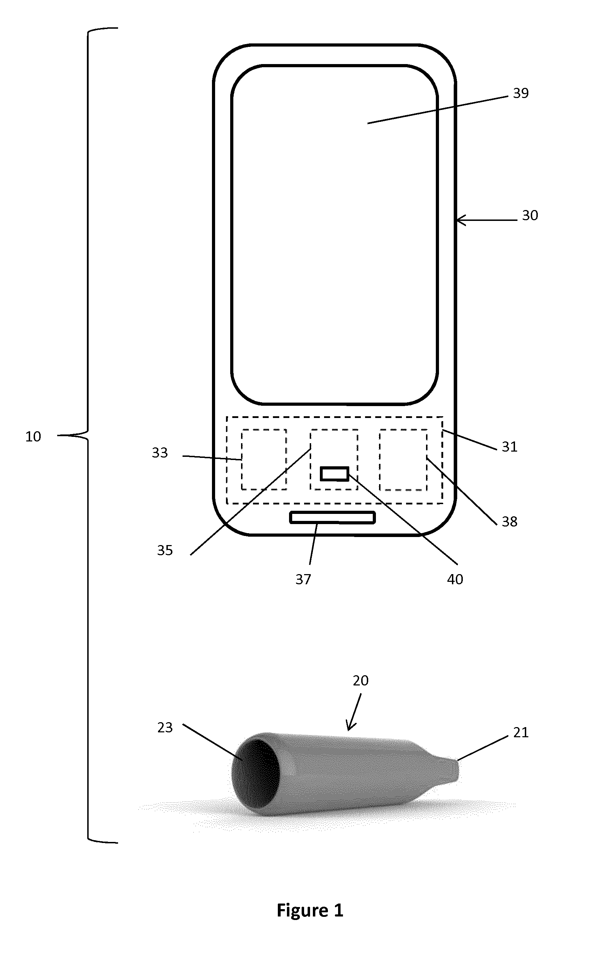

A system 10 for monitoring peak expiratory flow rate is shown schematically in Figure 1. The system 10 includes a peak expiratory flow rate monitoring device, which in this case is a whistle 20, and a separate detection device 30, such as a smart phone programmed with a monitoring application 40.

The whistle 20 is in this example a siren whistle (also known as a police whistle), and includes a mouthpiece 21 and a resonant chamber 23. Inside the whistle, between the resonant chamber and the mouthpiece, is a blade (not shown), arranged such that when air is blown through the mouthpiece onto the blade the airflow is destabilised and caused to resonate, so making a sound in a conventional manner.

The detection device 30 includes a control system 31 which comprises a processor 33 in signal communication with a memory 35, inside which the monitoring application 40 is stored. The detection device also includes a microphone 37, a display, and a user input device. In the example shown, the display and user input device are combined in a touchscreen display 39. Optionally, the detection device also include a transceiver 38 operable to transmit information, such as monitored data, from the device to a remote location, and to receive information, such as updates, from a remote location. As mentioned above, the detection device 30 may be a conventional smart phone loaded with a proprietary monitoring application 40.

It is known that peak expiratory flow rate can be estimated using a threshold-based whistle. Such threshold-based devices include a leak hole (or sometimes multiple leak holes) which can be varied in size (or covered and uncovered) so as to vary the resistance through the device. A larger leak hole means that a higher flow rate is needed through the device in order to produce a sound. Such devices typically require a patient to blow into the device multiple times, increasing the size of the leak hole each time. When a sound can no longer be produced, this is an indication that the patient's peak expiratory flow rate has been exceeded. The largest size of the leak hole at which the patient was able to produce a sound is thus indicative of the patient's peak expiratory flow rate.

A disadvantage of such devices is that they must be used multiple times in order to obtain a peak flow reading which is accurate. However, some patients, particularly those with severe asthma, are unable to perform repeated peak flow tests in quick succession. Furthermore, it can be difficult to tell when the threshold has been reached at low flow rates, or close to the patient's peak flow rate, because the sound produced by the whistle can be faint in these circumstances.

In contrast, the whistle 20 shown in Figure 1 is a conventional whistle having a fixed leak hole. That is, a leak hole which cannot be varied in size under normal use. The applicant has noted that the frequency of the sound produced by such a whistle varies with flow rate. In particular, a higher flow rate results in a higher frequency sound, whilst a lower flow rate results in a lower frequency sound.

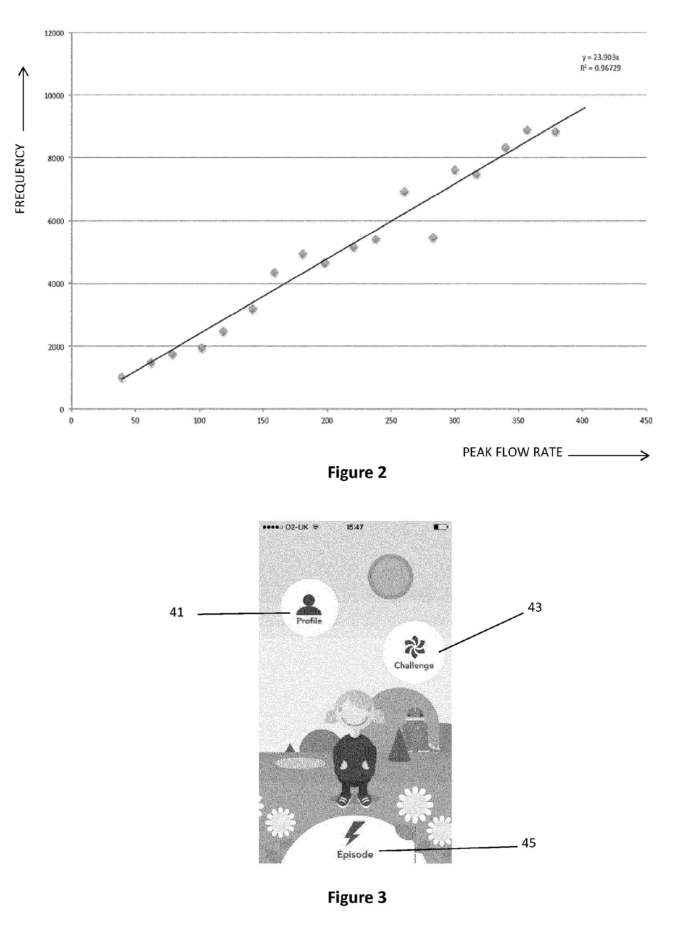

Figure 2 plots the relationship between peak flow rate (horizontal axis) and frequency (vertical axis) for the whistle in Figure 1. As can be seen, there is a clear (and in this case substantially linear) relationship between frequency and peak flow rate. The applicant has thus determined that a patient's peak expiratory flow rate can be monitored by detecting the frequency of the sound produced by a whistle which is blown into by the patient as part of a peak flow rate monitoring regime.

The applicant has developed a monitoring application 40 for use in conjunction with the peak flow monitoring whistle 20, which is simple to use and designed to improve patient compliance with daily monitoring of their asthma. It has been noted that patient compliance with an asthma monitoring regime is particularly poor amongst children, who can find the need to make daily peak flow measurements onerous. The embodiment of the applicant's monitoring application described herein is particularly designed to encourage children to comply with their asthma management plan. However, it is not limited to use by children.

An example graphic user interface of an asthma monitoring application 40 is shown in Figure 3. The interface incudes a profile button 41, a challenge button 43 and an incident report button 45. The profile button allows a user to view personal information about the patient's asthma history. The challenge button allows a user to initiate a challenge function and perform one or more peak expiratory flow rate tests. The incident report button allows a user to input data relating to the patient's asthma.

Another embodiment defaults to the peak flow challenge with the device listening for the whistle when the monitoring application is launched. This could be to reduce friction and immerse the user into the challenge experience as quickly as possible. A challenge button may nevertheless be provided on the home screen, in the event that the user chooses to exit the challenge, (e.g. to access another function).

In use, a patient can select the incident report button 45 (for example, by touching the region of the touchscreen display on which the "episode" icon is shown), and then use the touchscreen of the device to input asthma history data. It will be appreciated that the text on the buttons in the interface is for illustration purposes only.

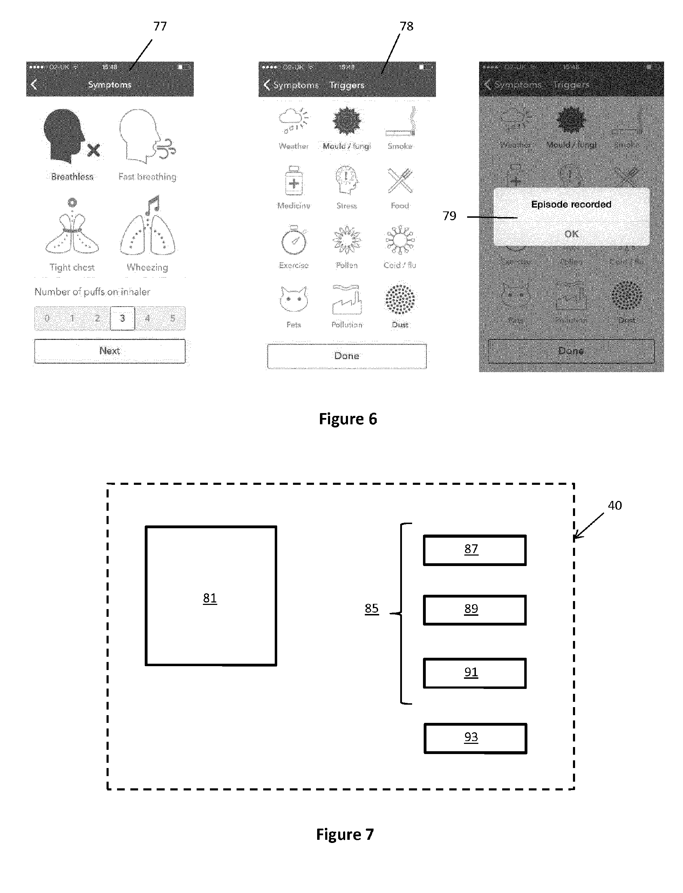

In this example, the patient (or another user) can use the incident report button to note that a patient has just experienced (or is experiencing) an asthma attack, as well as any triggers that may have caused the attack and any medication that was taken in order to control it. For example, when a user selects the incident report button he or she might be delivered to a first episode report screen 77, shown in Figure 6, in which the user is invited to log the symptoms associated with the attack being recorded by selecting the icons representing the symptoms experienced, such as breathlessness, fast breathing, tight chest or wheezing. The user may also be invited to indicate any medication taken in order to control the attack, for example, 1, 2, 3, 4 or 5 puffs on their inhaler, or zero puffs if the attack cleared without medication.

After entering their symptoms, the user might be delivered to a second incident report screen 78, in which the user is invited to log any triggers they noticed which could have been associated with the attack, such as weather, mould/fungi, smoke, medicine, stress, food, exercise, pollen, illness, pets, pollution or dust. When the attack has been successfully recorded in the device memory a completion icon 79 might be displayed.

The monitoring application may automatically record the time and date at which the incident is reported, and store this in the memory in association with the user input data. The application may also automatically record location-based environmental information including but not limited to pollen count, humidity, temperature or the presence of other allergens etc. Alternatively, the user may enter any or all of this data manually.

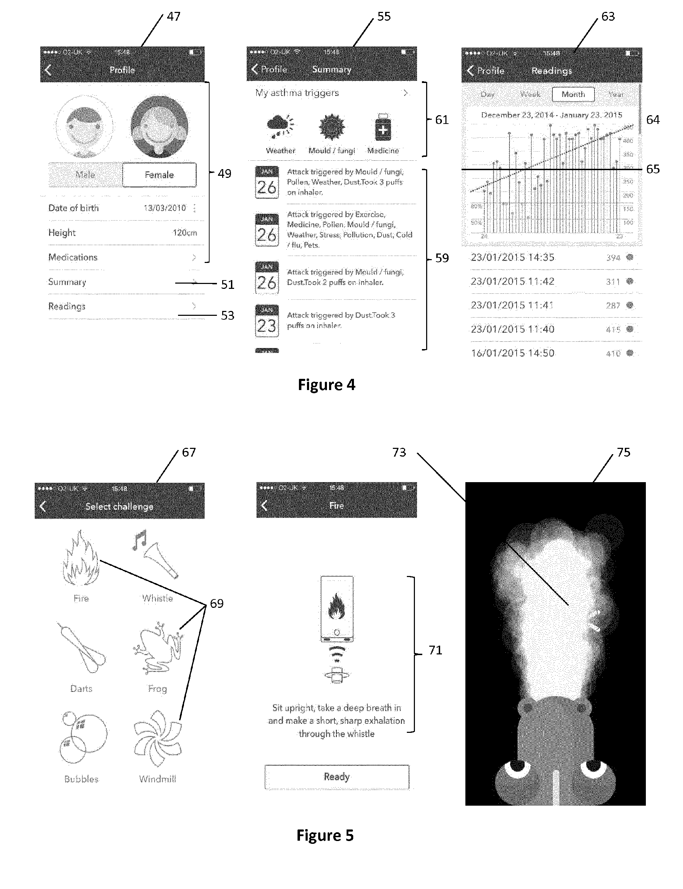

With reference to Figure 4, selecting the profile button 41 (for example, by touching the region of the touchscreen display on with the profile icon is shown), will deliver the user to a profile home screen 47. The profile home screen 47 incudes user information data 49, which is specific to the patient, for example the patient's sex, height, date of birth and any medications which they are prescribed. This information can be entered by the patient or their clinician using the touchscreen when setting up the patient's asthma management regime. This user data can be used by the application to determine an expected peak flow rate for the patient based on average peak flow rate data stored in the application's memory, as will be described in more detail below.

The profile home screen 47 also includes a summary button 51 and a readings button 53. When the summary button 51 is selected by a user (again, such as by touching the region of the touchscreen display on which the summary icon is shown), the user is delivered to a summary screen 55. The summary screen includes patient asthma history information 59 (as input by the patient using the incident report button), and trigger information 61, which may be extracted from the patient asthma history information by the application 40. Selectively displaying trigger information in a single location in addition to displaying it as part of the patient's history information helps to assist the patient (or their clinician) in identifying situations or substances which have been historically shown to trigger the patient's asthma.

When the readings button 53 is selected (such as by touching the region of the touchscreen display on which the readings icon is shown), the user is delivered to a readings screen 63. This screen displays historical peak expiratory flow rates detected by the device. This flow rate data is displayed in two formats, a list format setting out the date, time and value of the numerous detected flow rates, and a graph format which allows detected flow rates to be visually compared to an average flow rate 65 for the patient's age, sex and height, as well as to a trend line 64, which shows how the patient's own readings have altered over time. The readings (in both formats) are also colour coded to assist with the comparison. In particular, a detected flow rate which is 80 to 100 percent of the usual or normal peak flow readings (or more) is depicted in green, to indicate that no action is required. A detected flow rate which is 50 to 79 percent of the usual or normal peak flow readings is depicted in yellow to indicate that if such readings persist the patient's asthma may be worsening, and further medication/management steps may be required. A detected flow rate which is less than 50 percent of the usual or normal peak flow readings is depicted in red, indicating that medical attention may be needed.

In use, flow rate data is detected by the device using a challenge function of the application 40. On selecting the challenge button, a user is directed to a challenge screen 67. The challenge screen includes one or more challenge icons 69, each of which initiates a different challenge when its respective icon is selected. Each challenge is designed to encourage a user to correctly perform a peak flow expiratory flow rate test.

A correctly performed peak expiratory flow rate test requires the patient to sit upright, inhale deeply and exhale sharply, in order to obtain an indication of the patient's maximum speed of exhalation. A shallow inhalation or slow exhalation will result in an incorrect (usually low) reading. The example challenge shown in Figure 5 is provided in the form of a game including animation designed to encourage this type of exhalation. In particular, the challenge includes a first screen comprising instructions to begin the test, that is, to blow into the whistle, and a second screen providing a visual indication of the outcome of the peak flow rate test. The first screen, in this example, includes a motivational icon 71 instructions for performing a peak flow test. The second screen 75 in this case depicts an image of a dragon breathing fire. The second screen may also include a target icon or region 73. By posing the test as a game, the goal of the game being to cause the dragon to blow fire at the target region, a user is encouraged to perform a peak expiratory flow rate test to the best of their capability in order to do as well as possible at the game.

Further details of the monitoring application 40 are shown schematically in Figure 7. The monitoring application includes a control module 81 and stored data 85, which includes patient data 87, reference data 89 and calibration data 91. Patient data 87 includes the specific data entered into the application by the patient or their clinician, including user information data 49 and asthma history data 51. Reference data 87 includes stored generic peak expiratory flow rate data setting out average peak expiratory flow rates for a cross section of patients of various ages, sexes and heights. This data may be in any suitable form, for example a look up table or database. Calibration data 91 includes stored data detailing the relationship between peak expiratory flow rate and frequency for the whistle 20. This data may be in any suitable form, for example a mathematical relationship or a look up table.

When the monitoring application is in use, the control module 81 is operable to use the stored data 85 to generate patient peak expiratory flow rate data 93 using a frequency of the sound produced by the whistle 20 and detected by the microphone 37 of the detection device 30. In particular, the control module is operable to calibrate the detected frequency by comparing the detected frequency to the stored calibration data, so as to determine a peak expiratory flow rate for the test instance. The determined peak expiratory flow rate can then, if desired be compared with the reference data 89 and the patient data 87 in order to provide ranking information, for example in the form of a comparison of the determined flow rate with an expected average flow rate for healthy subjects of the same age, sex and height as the patient.

The monitoring application may also be operable to receive environmental data from a remote location, such as information on the weather or pollen count from a remote server, or from another application on the detection device (e.g. a weather information application). This information can be provided to the user, and/or stored in the device memory in association with patient data.

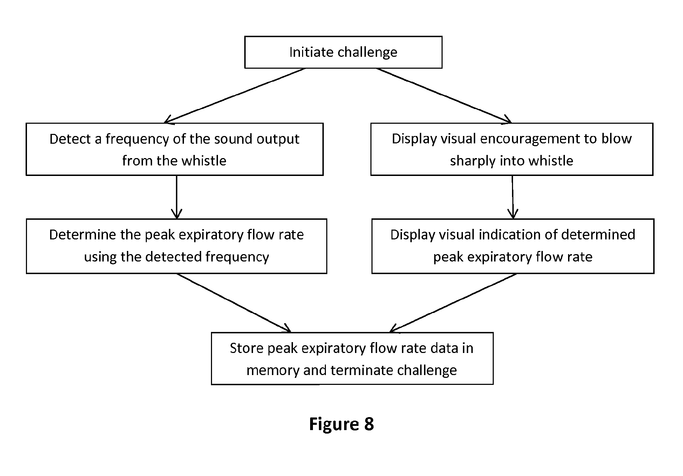

Referring now to Figure 8, a method of monitoring peak flow rate will be described. In use, a patient initiates a peak flow rate test by selecting a challenge icon 69 from the available option provided on the challenge screen 67. The control module then prompts the display of the detection device to present the patient with visual encouragement to blow sharply into the whistle 20. Simultaneously, the control module utilizes the microphone of the device to detect a frequency of sound output from the whistle. The control module then uses the detected frequency to determine the peak expiratory flow rate of the patient's exhalation, for example by comparing it with stored calibration data as discussed above.

Whilst sound is being detected in the microphone, a visual indication of the frequency of that sound is provided to the patient via the display of the detection device. For instance, in the example given of a dragon breathing fire, the amount of fire which is shown being produced by the dragon might be proportional to the detected flow rate (e.g., if a higher frequency is detected, more fire may be displayed than if a lower frequency is detected). The amount of fire displayed may correspond to the green, yellow and red rankings of the detected flow rate discussed above. For instance, if a detected peak flow rate is "green" (i.e. 80 to 100 percent of the usual or normal peak flow rate for the patient's height, sex and age), then the fire produced by the dragon may be indicated as reaching the target zone on the display. Conversely, if the detected flow rate is "yellow" (50 to 79 percent of the usual or normal peak flow rate) the fire may fall short of the target zone. If the detected flow rate is "red" (less than 50 percent of the usual or normal peak flow rate) then the dragon might produce no fire, or only a small amount of fire. In this way the patient is immediately provided with a simple visual indication of their peak flow rate, as well as with encouragement to improve their peak flow reading next time (to try and reach the target zone).

The control module may be operable to use the transceiver of the device to transmit the detected peak expiratory flow rate data 93 to a remote location, such as a computer of the patient's clinician or parents. This allows the patient's compliance with their asthma management plan to be monitored remotely if required.

The monitoring application could be arranged to provide the patient with an indication whether the peak expiratory flow rate test has been correctly performed, for example by analysing a change in frequency of the detected sound (if any) over the course of the test. For example, if a frequency of the detected sound increases during a test, this may be an indication that the patient was not initially blowing fast enough into the whistle, which indicates an incorrectly conducted test. In such instances a "try again" icon, or similar, could be displayed on the screen of the detection device.

The monitoring application could be further arranged to provide a confidence score associated with each test result. In order to provide as accurate a peak expiratory flow reading as possible, it is preferable to perform each challenge a plurality of times (for example 3-5 times) and then select the highest reading. The application could provide each flow rate with a confidence ranking based on the number of test instances performed, and/or the standard deviation of the test instances, and/or the frequency analysis of the test instances.

The foregoing method and system for monitoring peak expiratory flow rate is designed to improve the ease of asthma monitoring, and hence encourage compliance with an asthma management plan. The graphic user interface is simple to understand and visually interesting to children. Posing the test as a game makes it fun to use, providing motivation to take regular tests and improve lung function. Furthermore, the simple visual indication of flow rate can easily be understood even by young children, in contrast to current schemes requiring recordal of numerical values.

Although the invention has been described primarily with reference to monitoring of peak expiratory flow rate, the system can be used to monitor other characteristics of expiratory flow rate, for example, to perform spirometry. The system could also be used to assist a user in breathing exercises (e.g. for relaxation).

Although one example of a game has been described, it will be appreciated that many game variations are possible. For example a musical game could be provided based on the musical pitch of the detected whistle frequency.

The method does not require any proprietary equipment, and can be performed with an off the shelf whistle if desired. Although the system has been described with reference to a conventional siren whistle, it will be appreciated that other whistle types can be used, for example a slide whistle or kettle whistle. The whistle need not produce a musical note, but could instead produce a repetitive sound such as clicking. A "whistle" as defined herein thus refers to a device which produces a sound from a stream of forced air, wherein the frequency of the sound produced (which may be the apparent pitch, but could be regularity of clicking) is proportional to the air flow rate through the device.

It will be appreciated that the foregoing description sets out an example embodiment only, and that the scope of the invention is defined by the following claims.

## Claims

1. A method of measuring expiratory flow rate of a subject using a whistle, the method comprising:
detecting a sound generated when the subject blows into the whistle; and
determining the expiratory flow rate of the subject using a frequency of the detected sound.

2. The method of claim 1, wherein the detected frequency is compared to stored calibration data in order to determine the expiratory flow rate of the subject.

3. The method of claim 1 or claim 2, wherein the expiratory flow rate is a peak expiratory flow rate.

4. The method of claim 3, further comprising determining an expected peak flow rate for the subject using peak flow rate data and user information data.

5. The method of claim 3, further comprising comparing the detected peak expiratory flow rate of the subject with the expected peak expiratory flow rate in order to generate ranking data for the subject.

6. The method of any preceding claim, wherein the sound is detected in a detection device which is separate from the whistle.

7. The method of claim 6, wherein the detection device comprises a display, and wherein the method further comprises displaying a visual indication of the frequency of the detected sound on the display whilst the expiratory flow rate test is in progress.

8. The method of claim 6 or claim 7 when dependent on claim 5, wherein the detection device comprises a display, and wherein the method further comprises displaying a visual indication of the ranking data on the display whilst the peak expiratory flow rate test is in progress.

9. The method of any one of claims 6 to 8, further comprising storing the determined peak expiratory flow rate in a memory of the detection device.

10. The method of claim 9, further comprising associating environmental data with the determined peak expiratory flow rate, and storing the associated environmental data in the memory of the detection device.

11. The method of any one of claims 6 to 10, wherein the detection device comprises a transceiver, and wherein the method further comprises transmitting the determined peak expiratory flow rate to a remote location.

12. The method of any preceding claim, further comprising determining a plurality of values for the subject's peak expiratory flow rate in a single test session, selecting the highest of the plurality of values as the peak expiratory flow rate value for that test instance.

13. The method of any preceding claim further comprising generating calibration data, wherein the calibration data is generated by blowing air through the whistle at a plurality of known flow rates, and, for each flow rate, detecting a frequency of sound produced by the whistle at that flow rate.

14. The method of any preceding claim, further comprising generating an alarm indicating that a test is advised.

15. A computer program operable, when run on a detection device, to cause the detection device to operate as claimed in any of claims 1 to 14.
